# EUROPEAN PATENT APPLICATION

(11) **EP 4 703 368 A1**
(43) Date of publication of application: **04.03.2026**
(21) Application number: 24196406.3
(22) Date of filing: 26.08.2024
(51) Int. Cl.: C07J 41/00, A61P 11/00

(54) **ISOLITHOCHOLIC ACID DERIVATIVES AND USE THEREOF**

(71) Applicant: Instytut Biologii Doswiadczalnej PAN. im. M. Nenckiego, 02-093 Warszawa (PL)
(72) Inventor: Wypych, Tomasz, 00-740 Warszawa (PL); Bulanda, Edyta, 00-158 Warszawa (PL)
(74) Representative: JWP Patent & Trademark Attorneys

(57) **Abstract**

The subject matter of the invention is a compound of the formula (I), or a salt, prodrug, solvate, hydrate, or stereoisomer thereof, wherein Rₙ represents groups for n=1, 2, or 3, as follows R₁ represents -NHCH(CH₃)₂, R₂ represents -CONH₂, R₃ represents -CH₂NH₂. The another subject matter of the invention is a compound according to the present invention for use in a prevention and/or treatment of inflammatory diseases of the respiratory system and/or prevention and/or treatment of cytokine storm-associated diseases.

## Description

### Field of the invention

The present invention relates to isolithocholic acid derivatives and use thereof for prevention or treatment of inflammatory diseases of the respiratory system.

### Background

European patent EP3668879B1 discloses steroid compounds that have a formula represented by the following: (I) and wherein R1, R2, R3a, R3b, R4a, R4b, R5, R6a, R6b, R7, R8, and n are as described herein. The compounds may be prepared as pharmaceutical compositions, and may be used for promoting differentiation of T regulatory (Treg) lymphocytes, and for the prevention and treatment of a variety of conditions in mammals including humans, including by way of non-limiting example, inflammatory conditions, autoimmune disorders, and graft-versus-host disease.

International publication WO2020260558A1 discloses isolithocholic acid (3β-hydroxy-5β-cholan-24-oic acid) and isoallolithocholic acid (3β-hydroxy-5α-cholan-24-oic acid) together with the respective 22-homo-analogs or the deuterated analogs, which are modified in 3-position, for preventing or treating Clostridioides difficile-associated disease in a mammalian subject.

European patent application EP3004132A1 discloses compositions and methods for treating diseases or disorders associated with the metabolic syndrome using conjugates of bile acids with basic amino acids or a decarboxylated amino acid such as agmatine are provided. Further provided are bile acid- basic amino acid conjugates comprising chenodeoxycholic acid with an amino acid selected from arginine, lysine, histidine, ornithine or a decarboxylated amino acid such as agmatine.

Secondary bile acids are microbial products converted from primary bile acids. They have been shown to exert anti-inflammatory properties in the intestines through nuclear receptors and/or G protein-coupled receptors [1, 2]. Several bile acids were shown to be potent modulators of adaptive immunity. Lithocholic acid (LCA) inhibited the activation of Th1 cells in vitro via Vitamin D Receptor (VDR) signalling [3]. 3-oxo-lithocholic acid and isolithocholic acids inhibited Th17 differentiation in vitro and in vivo by binding RORyt, a master regulator of the Th17 subset, and inhibiting its transcriptional activity [4, 5]. Finally, isoallolithocholic acid (isoalloLCA) promoted the generation of mitochondrial reactive oxygen species, leading to enhanced expression of FOXP3 and differentiation of regulatory T cells in vitro [4, 6]. However, the potential impact of secondary bile acids on respiratory immunity has not been described. It is widely accepted that the gut microbiota profoundly shapes our immunity [7-9]. Dysbiosis in patients suffering from conditions characterized by airway inflammation has been described [10-14], which triggered interest in reducing symptoms of these diseases via probiotic administration [15, 16]. However, this approach raises three major concerns. First, probiotics fail to colonize the host, which prevents the long-lasting effect [17]. Second, as live organisms, probiotics adjust their metabolic functions according to new environments they are introduced to (different in terms of the microbiota/immune-metabolic states in each person), and thus, may lose their beneficial potential [18]. Finally, the concentrations of microbial products become diluted along the transit from the gut to the lungs (as demonstrated in the case of short-chain fatty acids [19, 20]), which might hinder their protective effect.

A recent SARS-Cov-2 pandemic illustrated an emerging medical need to develop therapeutic interventions to reduce airway pathology in patients experiencing airway inflammation. One strategy to achieve this outcome is the use of anti-inflammatory drugs. Despite extensive efforts, many clinical trials failed to prove the efficacy of candidate compounds. These include immunomodulators such as anti-interleukin-6 or anti-interleukin-1 monoclonal antibodies [21], hydrocortisone [22], azithromycin [23], doxycycline [24], interferon beta-1a [25], or the Janus kinase (JAK)1/JAK2 inhibitor ruxolitinib [26].

Several therapeutics did show a clinical benefit. These include dexamethasone [27], anti-IL-6R monoclonal antibody Tocilizumab [28], or Janus-associated tyrosine kinase (JAK) 1 and JAK 2 inhibitor, Baricitinib [29]. However, their efficacy is still limited (e.g. the improved mortality rate in the case of dexamethasone treatment is 22.9% vs 25.7% in the control group) [27]. In addition, those drugs are delivered orally or intravenously, which increases the likelihood of off-target effects. The primary mode of action behind these compounds relied on the effective inhibition of neutrophil influx, which is a hallmark of several respiratory diseases, including bacterial or viral infection-induced ARDS [30], neutrophilic severe asthma [31], chronic obstructive pulmonary disease (COPD) [32], bronchiectasis [33], or idiopathic pulmonary fibrosis (IPF) [34]. Of note, all of these mediators CXCL1, CCL2, CCL5, CXCL10, and IL-6 have been implicated in the pathogenesis of ARDS, neutrophilic asthma, COPD or IPF [31, 35-43].

Influx of neutrophils into the lung tissue is responsible for immunopathology in ARDS [30, 44, 45]. Moreover, major cause of the immunopathology of ARDS are carried by influx of dendritic cells and pro-inflammatory (Ly6C+) monocytes [46-50]. It is worth to mention that Cystatin C and clusterin, among other markers, have been recommended for use in preclinical development by the Predictive Safety Testing Consortium in collaboration with the US Food and Drug Administration (FDA), the European Medicines Agency and the Japanese Pharmaceuticals and Medical Devices Agency (PMDA) and the number of pharmaceutical companies [52].

Bile acids are known to be recognized by two classes of receptors, nuclear receptors (farnesoid X receptor, pregnane X receptor, vitamin D receptor, RAR-related orphan receptor gamma, the liver X receptor, and Nuclear Receptor Subfamily 4 Group A Member 1 and constitutive androstane receptor) and G protein-coupled receptors (sphingosine-1-phosphate receptor 2, Takeda G protein-coupled receptor 5, and muscarinic acetylcholine receptor M3) [53, 54].

Activation of a nuclear receptor, farnesoid X receptor (FXR) was shown to induce expression of a transcriptional repressor, SHP. In turn, SHP was demonstrated to interact with a transcription factor AP1, preventing AP1-driven expression of inflammatory genes [55], or directly bind to regulatory elements of inflammatory genes [56]. Activation of a G protein-coupled receptor, Takeda G protein-coupled receptor 5 (TGR5, also known as the G protein-coupled bile acid receptor 1, GPBAR1), was reported to activate protein kinase A (PKA) and cAMP-responsive element-binding protein (CREB), thereby reducing NF-κB activity in a STAT-1-dependent fashion [57-59]. Finally, both FXR and TGR5 were demonstrated to inhibit NLRP3 inflammasome activation. TGR5 signaling led to PKA-dependent phosphorylation of NLRP3, which resulted in NLRP3 ubiquitination and prevention of inflammasome assembly. FXR, on the other hand, physically interacted with caspase 1 and NLRP3, which prevented inflammasome assembly [53].

In light of the above, the approach to administer a microbial metabolite or its derivatives directly to the site of the interest overcomes problems with probiotic regimens in the following ways: i) administration of purified microbial compounds does not rely on microbial colonization; ii) the function of administered compounds is not a subject of adaptation to a new environment; iii) their safety is easier to ensure, and iv) the route along the gut-lung axis is bypassed via direct intranasal administration, maximizing bioavailability of the compound in the airways.

### Summary of the invention

The first aspect of the invention is a compound of the formula (I): 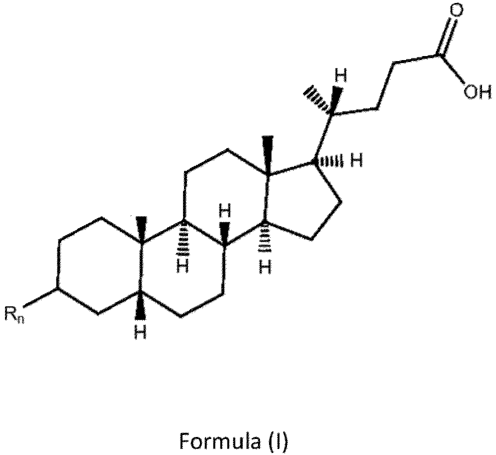 or a salt, prodrug, solvate, hydrate, or stereoisomer thereof, wherein Rₙ represents groups for n=1, 2, or 3, as follows:
R₁ represents -NHCH(CH₃)₂,
R₂ represents -CONH₂,
R₃ represents -CH₂NH₂.

In some embodiments the compound is selected from the group consisting of:

| Chemical structure | Chemical name | Structure number |
|---|---|---|
| 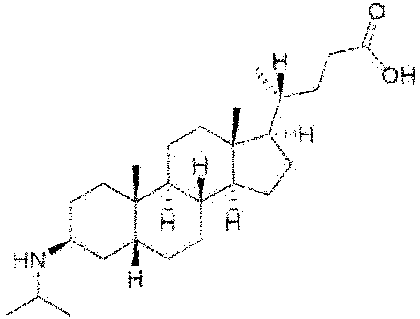 | (4R)-4-[(3R,5R,8R,9S,10S,13R,14S,17 R)-3-isopropylamine-10,13-dimethyl-2,3,4,5,6,7,8,9,11,12,14,15,16 ,17-tetradecahydro-1H-cyclopenta[a]phenanthren-17-yl]pentanoic acid | B1-3 |
| 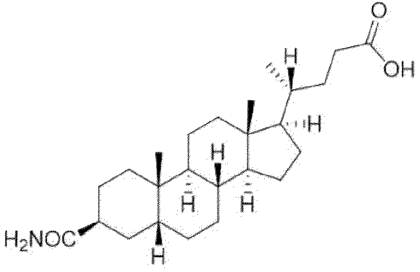 | (4R)-4-[(3R,5R,8R,9S,10S,13R,14S,17 R)-3-amide-10,13-dimethyl-2,3,4,5,6,7,8,9,11,12,14,15,16 ,17-tetradecahydro-1H-cyclopenta[a]phenanthren-17-yl]pentanoic acid | B1-X2 |
| 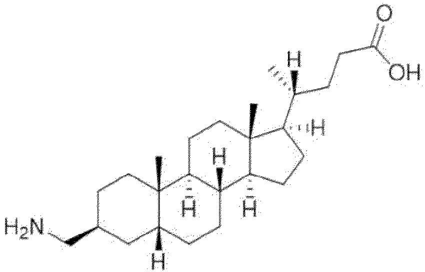 | (4R)-4-[(3R,5R,8R,9S,10S,13R,14S,17 R)-3-methylaminomethyl-10,13-dimethyl-2,3,4,5,6,7,8,9,11,12,14,15,16 ,17-tetradecahydro-1H-cyclopenta[a]phenanthren-17-yl]pentanoic acid | B1-X4 |

The second aspect of the invention is a composition comprising at least one compound or salt, prodrug, solvate, hydrate, or stereoisomer thereof.

In some embodiments a composition further comprises at least one carrier.

The third aspect of the invention is a compound according to the present invention for use in a prevention and/or treatment of inflammatory diseases of the respiratory system and/or prevention and/or treatment of cytokine storm-associated diseases.

In some embodiments a compound for use in the prevention and/or treatment of inflammatory diseases of the respiratory system by inhibition of neutrophil influx.

In some embodiments a compound for use in the prevention and/or treatment of inflammatory diseases of the respiratory system is based on inhibition of the production of one or more of the following cytokine: CXCL1, CCL2, CCL5, CXCL10, IL-6.

In some embodiments a compound is used in the inflammatory diseases of the respiratory system such as: ARDS, viral respiratory infections, bacterial respiratory infections, neutrophilic asthma, chronic obstructive pulmonary disease, bronchiectasis, idiopathic pulmonary fibrosis.

The particular advantage of our invention is a localized delivery in a form of a nasal spray and the capacity to inhibit a broad spectrum of inflammatory genes, which increases the efficacy of the treatment.

According to the invention, compounds disclosed in the invention may be used in many technical fields, especially in medicine, veterinary, pharmaceutical field mainly but not limited to the prevention or treatment of diseases, particularly inflammatory diseases of the respiratory system or cytokine storm-associated diseases.

According to the invention we propose isolithocholic (isoLCA) derivatives to be formulated as prophylactic and/or therapeutic formulations against respiratory conditions characterized by neutrophilia, including but not limited to following disorders: ARDS, viral or bacterial respiratory infections, neutrophilic asthma, chronic obstructive pulmonary disease, bronchiectasis or idiopathic pulmonary fibrosis.

### Brief description of drawings

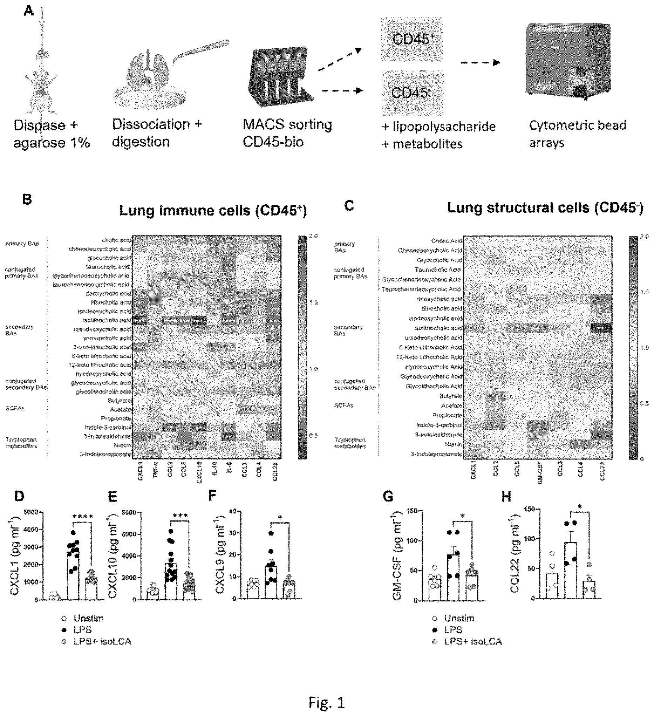

Fig. 1 IsoLCA profoundly inhibits the pro-inflammatory response in lung cells. A) Experimental setup. Primary murine lung cells were isolated using dispase II and 1% agarose solution and MACS-sorted into CD45+ and CD45- fractions. Cells were then stimulated with LPS (10 ng/ml) in the presence of tested metabolites, and the pro-inflammatory response was assessed using cytometric bead arrays. B) Lung Immune cells (CD45+) and C) Lung structural cells (CD45-) were stimulated with LPS (10 ng/ml) in the presence of tested metabolites. The pro-inflammatory response was assessed by cytometric bead arrays and compared to control conditions (cells stimulated with LPS in the presence of a vehicle). D), E), F) Select analytes (<0.5 fold change) from the immune cell compartment. G, H) Select analytes (<0.5 fold change) from the structural cell compartment.
Fig. 2 presents the activity of LCA, bearing the opposite orientation of the C3-hydroxyl group (at the concentration of 50 µM), being a base to synthesise 21 derivatives of isoLCA.

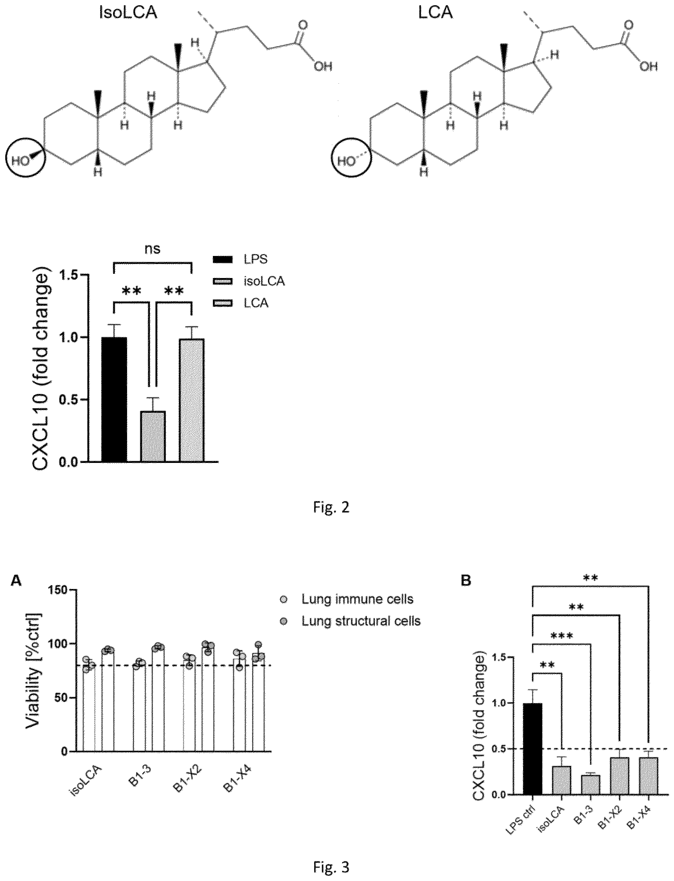

Fig. 3 presents compounds B1-3, B1-X2, and B1-X4 (Table 1) retain the anti-inflammatory potential of isoLCA. A) Cellular toxicity assay of 3 isoLCA derivatives (at 50 µM). Equal volumes of PBS or medium and CellTiter-Glo reagent were added per well with immune lung cells or structural lung cells. After 2 minutes of incubation in a thermomixer at room temperature, 800 rpm luminescence was measured using Tecan M1000PRO. B) Activity of these derivatives of isoLCA (below 80% of live control) (at 50 µM). Data points represent biological replicates pooled from 3 independent experiments. Error bars represent the SEM. *p<0.05, **p<0.01.

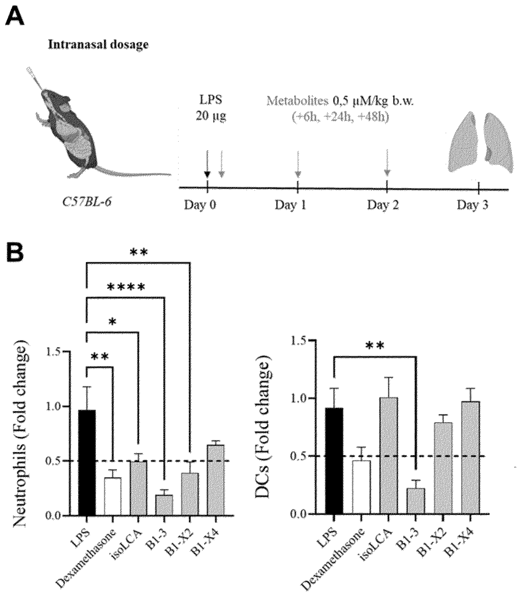

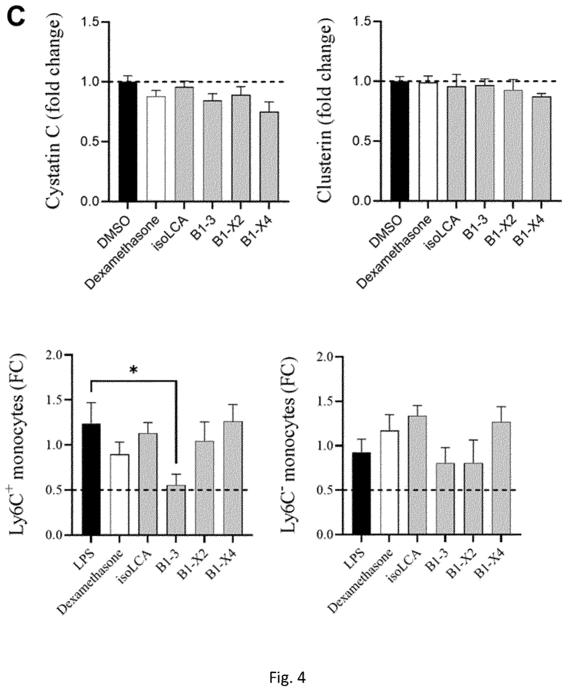

Fig. 4 presents the therapeutic potential of isoLCA, B1-3, and B1-X2 (Table 1) in a mouse model of ARDS; A) Experimental setup. C57BL/6J mice were administered with 20 µg of LPS intranasally. isoLCA, B1-3, B1-X2, or B1-X4 (Table 1) were administered intranasally 6 hours, 24 hours, and 48 hours later at the dose of 0,5 µM/kg body weight. Lungs were collected on day 3; B) Numbers of neutrophils, dendritic cells, Ly6C⁺, and Ly6C⁻ monocytes were obtained with the use of flow cytometry and calculated as fold change in comparison to the control group (administration of LPS followed by the vehicle treatment); C) Concentrations of Cystatin C and Clusterin were measured by ELISA and calculated as fold change to the control (DMSO). Data points represent 8 biological replicates pooled from 2 independent experiments. Error bars represent the SEM. *p<0.05, **p<0.01, ****p<0.0001,

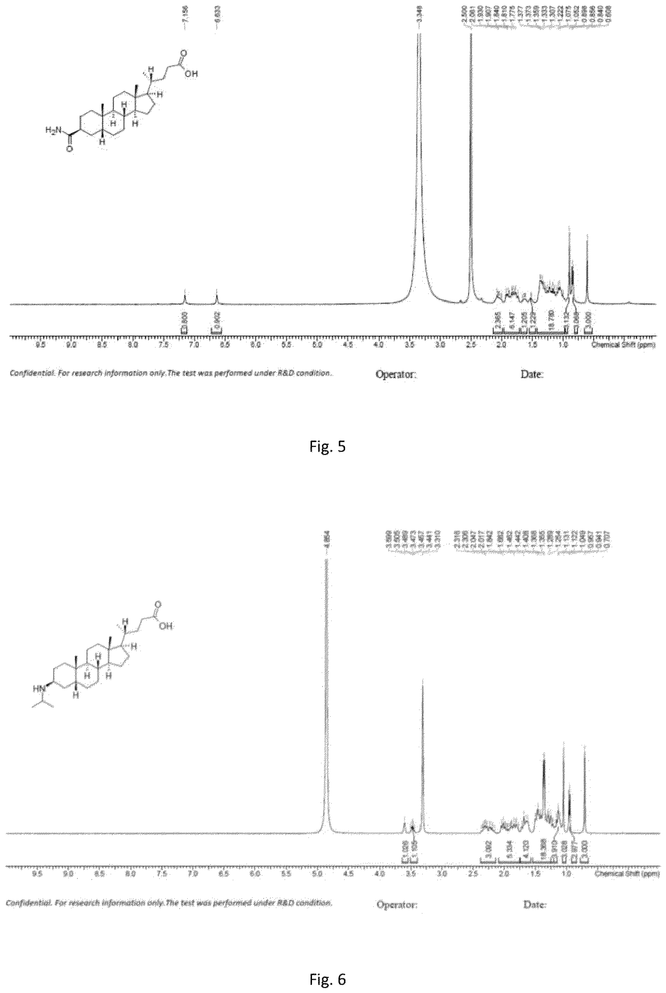

Fig. 5 presents H¹NMR spectrum of B1-X2 compound,
Fig. 6 presents H¹NMR spectrum of B1-3 compound,

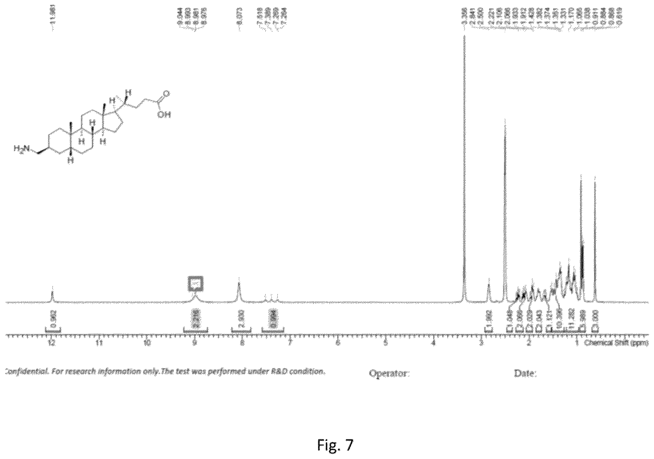

Fig. 7 presents H¹NMR spectrum of B1-X4 compound.

### Embodiments

Compound of lithocholic acid in amount of 1 eq and thionyl chloride in amount of 2 eq were dissolved in anhydrous methanol (10 V; 10 x volume of reagents) and stirred for 2 hours in 70°C. Protected lithocholic acid was separated and purified. Reaction of compound (3) in amount of 1 eq with chloromesylate in amount of 2 eq and triethyloamine in amount of 3 eq was carried out in dichloromethane (30 V) at 0°C for 2 hours in quantitative yield. Procedure was carried out before all synthesis of the invention.

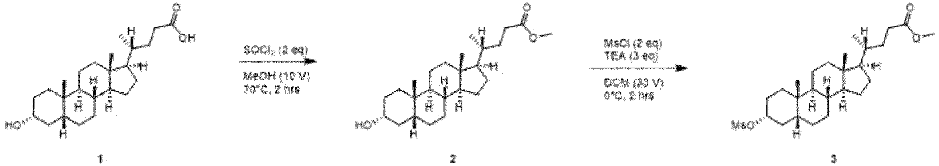

### Embodiment 1

Isolated and purified compound (3) underwent substitution with NaCN in THF at 65°C for 24 hours, resulting in compound (4). Sodium hydroxide and methanol in water solution were added to the mixture, yielding compound Cpd B1-X2.

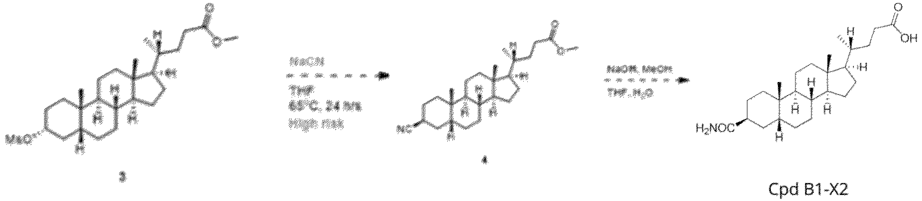

### Embodiment 2

Isolated and purified compound (3) underwent reaction with isopropylamine in N-methyl-2-pyrrolidone (NMP) at 120°C for 12 hours in sealed tube. Sodium hydroxide and methanol in water solution were added to the mixture, yielding compound Cpd B1-3.

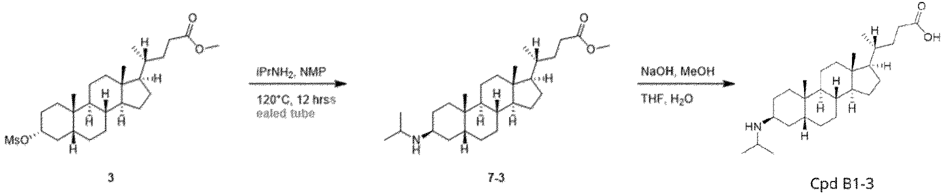

### Embodiment 3

Isolated and purified compound (3) underwent substitution with NaCN in DMF at 80°C for 20 hours, resulting in compound (4). Isolated compound (4) was subjected to hydrogenation process using catalyst Pd/C in EtOH, AcOH at 80°C for 12 hours. Ester hydrolysis was done using sodium hydroxide, resulting in compound Cpd B1-X4.

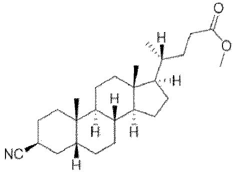

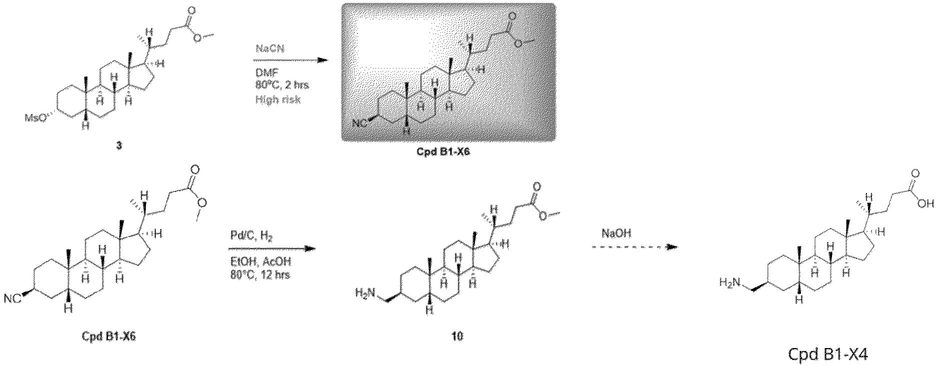

The obtained compounds namely Cpd B1-X2, Cpd B1-3, Cpd B1-X4 were in the form of white solids with a purity of ≥97%.

### Embodiment 4 - Inhibition properties of the pro-inflammatory response in the lungs

We screened the library of metabolites for their capacity to inhibit a panel of inflammatory mediators that are typically observed during acute respiratory distress syndrome. For this, we used lungs isolated from naive mice C57BL/6J bred in the animal house at our Institute, and after using MACS sorting (Miltenyi Biotec), we obtained CD45 positive cells. We stimulated them with LPS (1 µg/ml), which mimics the inflammation in the presence or absence of metabolites in 50 µM concentration, established as non-toxic. After 24h, we collected the supernatant, and the production of inflammatory mediators was analysed by cytometric bead arrays (Legend Plex) or ELISA (R&D or Invitrogen). By stimulating lung immune cells with lipopolysaccharide (a compound that mimics a bacterial infection), we found isoLCA to be the most effective among tested compounds at inhibiting the proinflammatory response. It inhibits the production of CXCL1, CCL2, CCL5, CXCL10 and IL-6 to a statistically significant degree (fold change <0,5 versus LPS alone) (Fig. 1). Based on this, we decided to synthesise 21 derivatives of isoLCA, since we noted that its natural isomer, lithocholic acid (LCA), which differs in the three-dimensional orientation of the C3-hydroxyl group, lost the immunosuppressive potential (Figure 2). This data indicated that the C3-hydroxyl group is important for the activity of isoLCA and suggested that its modification can alter the magnitude of the effect. We screened those compounds; 8 of them were non-toxic for lung cells (Fig.3A), and 3 of them showed immunosuppressive potential on lung cells even stronger than isoLCA (Fig.3B).

### Embodiment 5 - Protection properties against ARDS

To test whether identified compounds hold the potential to be developed as drugs against ARDS, we validated our findings in a mouse model of ARDS. C57BL/6J mice bred in the animal house at our Institute were administered with lipopolysaccharide from Escherichia coli O127:B8 (SigmaAldrich) intranasally (20 µg), and isoLCA (Cayman) and its derivatives were administered intranasally 6 hours, 24 hours, and 48 hours later at a dose of 0.5 µM/kg body weight (Fig. 4). After 3 days, mice were sacrificed, and cell infiltration in the lung and cytokine levels in BALF fluid were measured. (Fig. 4A). B1-3, and B1-X2 (Table 1) potently inhibited the influx of neutrophils into the lung tissue. Notably, B1-3 (Table 1) also inhibited the influx of dendritic cells and pro-inflammatory (Ly6C+) monocytes (Fig. 4B).

None of the compounds affected the population of patrolling monocytes, which are involved in removing damaged cells and wound healing (defined as Ly6C-[51]).

Finally, none of the compounds elevated the levels of kidney toxicity markers, cystatin C and clusterin (Fig. 4C). Together, these data indicate the therapeutic potential of B1-3, and B1-X2 (Table 1) in the context of ARDS.

### Embodiment 6 - Mechanism of action

In our laboratory, we have indicated an additional, previously undescribed mechanism by which isolithocholic acid boosts mitochondrial function in inflammatory settings. In the context of isoLCA derivatives, we discovered that they can suppress the production of chemokines recruiting neutrophils, reducing inflammation.

### References:

1. Wahlstrom, A., et al., Intestinal Crosstalk between Bile Acids and Microbiota and Its Impact on Host Metabolism. Cell Metab, 2016. 24(1): p. 41-50.
2. Fiorucci, S. and E. Distrutti, Bile Acid-Activated Receptors, Intestinal Microbiota, and the Treatment of Metabolic Disorders. Trends Mol Med, 2015. 21(11): p. 702-714.
3. Pols, T.W.H., et al., Lithocholic acid controls adaptive immune responses by inhibition ofTh1 activation through the Vitamin D receptor. PLoS One, 2017. 12(5): p. e0176715.
4. Hang, S., et al., Bile acid metabolites control TH17 and Treg cell differentiation. Nature, 2019. 576(7785): p. 143-148.
5. Paik, D., et al., Human gut bacteria produce TauEta17-modulating bile acid metabolites. Nature, 2022. 603(7903): p. 907-912.
6. Tanoue, T., et al., A defined commensal consortium elicits CD8 T cells and anti-cancer immunity. Nature, 2019. 565(7741): p. 600-605.
7. Ivanov, II and K. Honda, Intestinal commensal microbes as immune modulators. Cell Host Microbe, 2012. 12(4): p. 496-508.
8. Wypych, T.P. and B.J. Marsland, Antibiotics as Instigators of Microbial Dysbiosis: Implications for Asthma and Allergy. Trends Immunol, 2018. 39(9): p. 697-711.
9. Wypych, T.P., L.C. Wickramasinghe, and B.J. Marsland, The influence of the microbiome on respiratory health. Nat Immunol, 2019. 20(10): p. 1279-1290.
10. Zuo, T., et al., Alterations in Gut Microbiota of Patients With COVID-19 During Time of Hospitalization. Gastroenterology, 2020. 159(3): p. 944-955 e8.
11. Yeoh, Y.K., et al., Gut microbiota composition reflects disease severity and dysfunctional immune responses in patients with COVID-19. Gut, 2021. 70(4): p. 698-706.
12. Barcik, W., et al., The Role of Lung and Gut Microbiota in the Pathology of Asthma. Immunity, 2020. 52(2): p. 241-255.
13. Hufnagl, K., et al., Dysbiosis of the gut and lung microbiome has a role in asthma. Semin Immunopathol, 2020. 42(1): p. 75-93.
14. Li, N., et al., Gut microbiota dysbiosis contributes to the development of chronic obstructive pulmonary disease. Respir Res, 2021. 22(1): p. 274.
15. Hussain, I., et al., Role of Gut Microbiome in COVID-19: An Insight Into Pathogenesis and Therapeutic Potential. Front Immunol, 2021. 12: p. 765965.
16. Mortaz, E., et al., Probiotics in the management of lung diseases. Mediators Inflamm, 2013. 2013: p. 751068.
17. Zmora, N., et al., Personalized Gut Mucosal Colonization Resistance to Empiric Probiotics Is Associated with Unique Host and Microbiome Features. Cell, 2018. 174(6): p. 1388-1405 e21.
18. Suez, J., et al., The pros, cons, and many unknowns of probiotics. Nat Med, 2019. 25(5): p. 716-729.
19. Yue, M., et al., Measurement of Short-Chain Fatty Acids in Respiratory Samples: Keep Your Assay above the Water Line. Am J Respir Crit Care Med, 2020. 202(4): p. 610-612.
20. Cummings, J.H., et al., Short chain fatty acids in human large intestine, portal, hepatic and venous blood. Gut, 1987. 28(10): p. 1221-7.
21. Declercq, J., et al., Effect of anti-interleukin drugs in patients with COVID-19 and signs of cytokine release syndrome (COV-AID): a factorial, randomised, controlled trial. Lancet Respir Med, 2021. 9(12): p. 1427-1438.
22. Dequin, P.F., et al., Effect of Hydrocortisone on 21-Day Mortality or Respiratory Support Among Critically III Patients With COVID-19: A Randomized Clinical Trial. JAMA, 2020. 324(13): p. 1298-1306.
23. Oldenburg, C.E., et al., Effect of Oral Azithromycin vs Placebo on COVID-19 Symptoms in Outpatients With SARS-CoV-2 Infection: A Randomized Clinical Trial. JAMA, 2021. 326(6): p. 490-498.
24. Butler, C.C., et al., Doxycycline for community treatment of suspected COVID-19 in people at high risk of adverse outcomes in the UK (PRINCIPLE): a randomised, controlled, open-label, adaptive platform trial. Lancet Respir Med, 2021. 9(9): p. 1010-1020.
25. Kalil, A.C., et al., Efficacy of interferon beta-1a plus remdesivir compared with remdesivir alone in hospitalised adults with COVID-19: a double-bind, randomised, placebo-controlled, phase 3 trial. Lancet Respir Med, 2021. 9(12): p. 1365-1376.
26. Han, M.K., et al., Ruxolitinib in addition to standard of care for the treatment of patients admitted to hospital with COVID-19 (RUXCOVID): a randomised, double-blind, placebo-controlled, phase 3 trial. Lancet Rheumatol, 2022. 4(5): p. e351-e361.
27. Group, R.C., et al., Dexamethasone in Hospitalized Patients with Covid-19. N Engl J Med, 2021. 384(8): p. 693-704.
28. Writing Committee for the, R.-C.A.P.I., et al., Long-term (180-Day) Outcomes in Critically III Patients With COVID-19 in the REMAP-CAP Randomized Clinical Trial. JAMA, 2023. 329(1): p. 39-51.
29. Marconi, V.C., et al., Efficacy and safety of baricitinib for the treatment of hospitalised adults with COVID-19 (COV-BARRIER): a randomised, double-blind, parallel-group, placebo-controlled phase 3 trial. Lancet Respir Med, 2021. 9(12): p. 1407-1418.
30. Yang, S.C., et al., Understanding the role of neutrophils in acute respiratory distress syndrome. Biomed J, 2021. 44(4): p. 439-446.
31. Zhang, X., et al., The onset, development and pathogenesis of severe neutrophilic asthma. Immunol Cell Biol, 2022. 100(3): p. 144-159.
32. Hoenderdos, K. and A. Condliffe, The neutrophil in chronic obstructive pulmonary disease. Am J Respir Cell Mol Biol, 2013. 48(5): p. 531-9.
33. Summers, C., Chasing the "Holy Grail": Modulating Neutrophils in Inflammatory Lung Disease. Am J Respir Crit Care Med, 2019. 200(2): p. 131-132.
34. Mincham, K.T., et al., Our evolving view of neutrophils in defining the pathology of chronic lung disease. Immunology, 2021. 164(4): p. 701-721.
35. Chu, H., et al., Comparative Replication and Immune Activation Profiles of SARS-CoV-2 and SARS-CoV in Human Lungs: An Ex Vivo Study With Implications for the Pathogenesis of COVID-19. Clin Infect Dis, 2020. 71(6): p. 1400-1409.
36. Del Valle, D.M., et al., An inflammatory cytokine signature predicts COVID-19 severity and survival. Nat Med, 2020. 26(10): p. 1636-1643.
37. Zaid, Y., et al., Chemokines and eicosanoids fuel the hyperinflammation within the lungs of patients with severe COVID-19. J Allergy Clin Immunol, 2021. 148(2): p. 368-380 e3.
38. Harrison, C., Focus shifts to antibody cocktails for COVID-19 cytokine storm. Nat Biotechnol, 2020. 38(8): p. 905-908.
39. Santa Cruz, A., et al., Interleukin-6 Is a Biomarker for the Development of Fatal Severe Acute Respiratory Syndrome Coronavirus 2 Pneumonia. Front Immunol, 2021. 12: p. 613422.
40. Lai, C., et al., C-C Motif Chemokine Ligand 2 (CCL2) Mediates Acute Lung Injury Induced by Lethal Influenza H7N9 Virus. Front Microbiol, 2017. 8: p. 587.
41. Williams, A.E., et al., Evidence for chemokine synergy during neutrophil migration in ARDS. Thorax, 2017. 72(1): p. 66-73.
42. Chung, K.F. and I.M. Adcock, Multifaceted mechanisms in COPD: inflammation, immunity, and tissue repair and destruction. Eur Respir J, 2008. 31(6): p. 1334-56.
43. Schupp, J.C., et al., Macrophage activation in acute exacerbation of idiopathic pulmonary fibrosis. PLoS One, 2015. 10(1): p. e0116775.
44. Grommes, J. and O. Soehnlein, Contribution of neutrophils to acute lung injury. Mol Med, 2011. 17(3-4): p. 293-307.
45. Zemans, R.L. and M.A. Matthay, What drives neutrophils to the alveoli in ARDS? Thorax, 2017. 72(1): p. 1-3.
46. Dhaliwal, K., et al., Monocytes control second-phase neutrophil emigration in established lipopolysaccharide-induced murine lung injury. Am J Respir Crit Care Med, 2012. 186(6): p. 514-24.
47. Maus, U., et al., The role of CC chemokine receptor 2 in alveolar monocyte and neutrophil immigration in intact mice. Am J Respir Crit Care Med, 2002. 166(3): p. 268-73.
48. Mo, J., et al., Single-cell analysis reveals dysregulated inflammatory response in peripheral blood immunity in patients with acute respiratory distress syndrome. Front Cell Dev Biol, 2023. 11: p. 1199122.
49. Li, X.C., M. Miyasaka, and T.B. Issekutz, Blood monocyte migration to acute lung inflammation involves both CD11/CD18 and very late activation antigen-4-dependent and independent pathways. J Immunol, 1998. 161(11): p. 6258-64.
50. Li, L., et al., Classical dendritic cells regulate acute lung inflammation and injury in mice with lipopolysaccharide-induced acute respiratory distress syndrome. Int J Mol Med, 2019. 44(2): p. 617-629.
51. Thomas, G., et al., Nonclassical patrolling monocyte function in the vasculature. Arterioscler Thromb Vasc Biol, 2015. 35(6): p. 1306-16.
52. Campion, S., et al., The current status of biomarkers for predicting toxicity. Expert Opin Drug Metab Toxicol, 2013. 9(11): p. 1391-408.
53. Fiorucci, S., et al., Bile Acids Activated Receptors Regulate Innate Immunity. Front Immunol, 2018. 9: p. 1853.
54. Godlewska, U., E. Bulanda, and T.P. Wypych, Bile acids in immunity: Bidirectional mediators between the host and the microbiota. Front Immunol, 2022. 13: p. 949033.
55. Fiorucci, S., et al., The nuclear receptor SHP mediates inhibition of hepatic stellate cells by FXR and protects against liver fibrosis. Gastroenterology, 2004. 127(5): p. 1497-512.
56. Yang, Z., A.N. Koehler, and L. Wang, A Novel Small Molecule Activator of Nuclear Receptor SHP Inhibits HCC Cell Migration via Suppressing CcI2. Mol Cancer Ther, 2016. 15(10): p. 2294-2301.
57. Haselow, K., et al., Bile acids PKA-dependently induce a switch of the IL-10/IL-12 ratio and reduce proinflammatory capability of human macrophages. J Leukoc Biol, 2013. 94(6): p. 1253-64.
58. Biagioli, M., et al., The Bile Acid Receptor GPBAR1 Regulates the M1/M2 Phenotype of Intestinal Macrophages and Activation of GPBAR1 Rescues Mice from Murine Colitis. J Immunol, 2017. 199(2): p. 718-733.
59. Perino, A., et al., TGR5 reduces macrophage migration through mTOR-induced C/EBPbeta differential translation. J Clin Invest, 2014. 124(12): p. 5424-36.

## Claims

1. A compound of the formula (I): 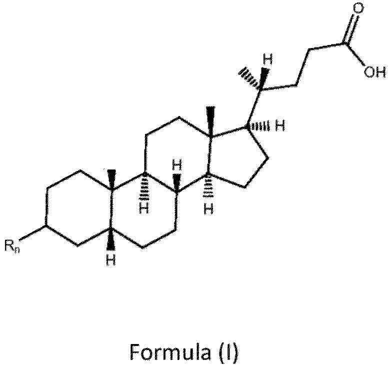 or a salt, prodrug, solvate, hydrate, or stereoisomer thereof, wherein Rₙ represents groups for n=1, 2, or 3, as follows:
R₁ represents -NHCH(CH₃)₂,
R₂ represents -CONH₂,
R₃ represents -CH₂NH₂.

2. The compound according to claim 1, **characterized in that** the compound is selected from the group consisting of:
| | |
|---|---|
| | |
| 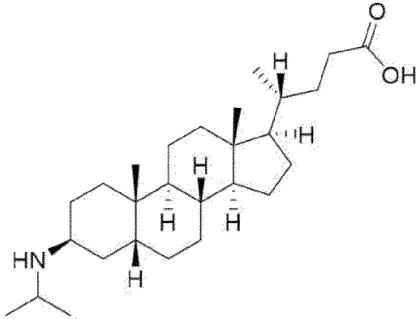 | (4R)-4-[(3R,5R,8R,9S,10S,13R,14S,17R)-3-isopropylamine-10,13-dimethyl-2,3,4,5,6,7,8,9,11,12,14,15,16,17 -tetradecahydro-1H-cyclopenta[a]phenanthren-17-yl]pentanoic acid |
| 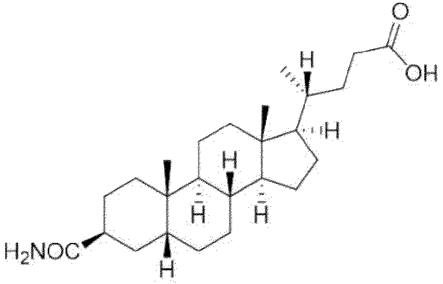 | (4R)-4-[(3R,5R,8R,9S,10S,13R,14S,17R)-3-amide-10,13-dimethyl-2,3,4,5,6,7,8,9,11,12,14,15,16,17 -tetradecahydro-1H-cyclopenta[a]phenanthren-17-yl]pentanoic acid |
| 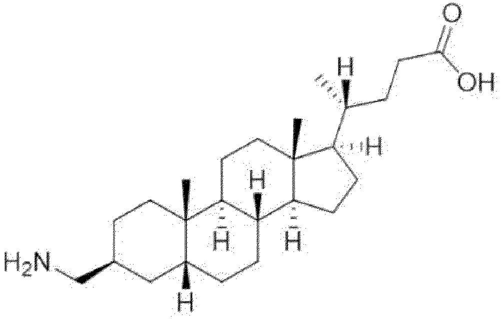 | (4R)-4-[(3R,5R,8R,9S,10S,13R,14S,17R)-3-methylaminomethyl-10,13-dimethyl-2,3,4,5,6,7,8,9,11,12,14,15,16,17 -tetradecahydro-1H-cyclopenta[a]phenanthren-17-yl]pentanoic acid |

3. A composition comprising at least one compound or salt, prodrug, solvate, hydrate, or stereoisomer thereof as defined in any one of claims 1-2.

4. A composition according to claim 3, further comprising at least one carrier.

5. A compound as defined in any one of claims 1-2 for use in the prevention and/or treatment of inflammatory diseases of the respiratory system and/or prevention and/or treatment of cytokine storm-associated diseases.

6. A compound for use according to claim 5, wherein prevention and/or treatment of inflammatory diseases of the respiratory system by inhibition of neutrophil influx.

7. A compound for use according to any one of claims 5-6, **characterised in that**, the prevention and/or treatment of inflammatory diseases of the respiratory system is based on inhibition of the production of one or more of the following cytokine: CXCL1, CCL2, CCL5, CXCL10, IL-6.

8. A compound for use according to any one of claims 5-7, wherein inflammatory disease of the respiratory system belongs to such group of instances as: ARDS, viral respiratory infections, bacterial respiratory infections, neutrophilic asthma, chronic obstructive pulmonary disease, bronchiectasis, idiopathic pulmonary fibrosis.
